# EUROPEAN PATENT APPLICATION

(11) **EP 3 892 275 A1**
(43) Date of publication of application: **13.10.2021**
(21) Application number: 20315131.1
(22) Date of filing: 08.04.2020
(51) Int. Cl.: A61K 31/4706, A61P 31/14

(54) **AEROSOLIZATION OF HCQ OR ITS METABOLITES FOR THE TREATMENT OF LUNG INFECTIONS**

(71) Applicant: INSERM (Institut National de la Santé et de la Recherche Médicale), 75013 Paris (FR); CENTRE NATIONAL DE LA RECHERCHE SCIENTIFIQUE (CNRS), 75016 Paris (FR); Centre Hospitalier Régional Universitaire de Nancy (CHRU), 54000 Nancy (FR); Université de Lorraine, 54000 Nancy (FR)
(72) Inventor: The designation of the inventor has not yet been filed
(74) Representative: Inserm Transfert

(57) **Abstract**

Coronaviruses cause severe diseases mainly of the respiratory and gastrointestinal tract. The infection of humans with coronaviruses have been known since the sixties to be associated with respiratory tract i.e. common cold-like diseases. Severe Acute Respiratory Syndrome-Corona Virus (SARS-CoV) is a highly aggressive in humans with often fatal outcome. The inventors aim at developing a new prophylactic or preventive therapeutic approach as well as a curative therapeutic approach based on the local delivery of hydroxychloroquine (HCQ) or its metabolite (e.g. Desethylhydroxychloroquine (DHCQ)) into the lung by aerosolization. The present invention relates to a method of treating Severe Acute Respiratory Syndrome-Corona Virus (SARS-CoV) in a patient in need thereof comprising administering to the patient's lungs a therapeutically effective amount of hydroxychloroquine (HCQ) and/or Desethylhydroxychloroquine (DHCQ) by aerosolization.

## Description

### FIELD OF THE INVENTION:

The present invention is in the field of infectiology. More particularly, the invention relates to methods and compositions for treatment of Severe Acute Respiratory Syndrome-Corona Virus (SARS-CoV) infection.

### BACKGROUND OF THE INVENTION:

Coronaviruses cause severe diseases mainly of the respiratory and gastrointestinal tract. The infection of humans with coronaviruses have been known since the sixties to be associated with respiratory tract i.e. common cold-like diseases. Severe Acute Respiratory Syndrome-Corona Virus (SARS-CoV) is a highly aggressive in humans with often fatal outcome. In late December 2019, a new betacoronavirus SARS-CoV-2 has emerged in Wuhan China. The World Health Organization has named the severe pneumonia caused by this new coronavirus COVID-19 (for Corona Virus Disease 2019, WHO, 2020). Since its emergence, the COVID-19 has spread to 159 countries across the five continents.

Thus, the COVID-19 pandemic constitutes a major problem for all health systems, with its ongoing extension and possible resurgences, and while considering current mortality, as well as potential morbidity due to fibrotic sequels. Fortunately, there is a mobilization on a scale never before attained worldwide for medical research and with an extensive number of clinical trials conducted on new treatments. Vaccine are being tested in healthy volunteers, but large-scale vaccination cannot be expected before 1 or 2 years.

Most of the tested antiviral treatments, and especially hydroxychloroquine (HCQ), are highly promising, at least on the basis preclinical and pilot studies. The oral administration of hydroxychloroquine (HCQ) has raised significant hopes, due to studies carried out at a cellular scale (1,2) but also, to promising results from pilot clinical studies (3,4). These data are still too scarce to lead to a formal indication and thus, to change medical practices. However, they have made possible to integrate the HCQ oral treatment in many randomized clinical trials planned worldwide, at various stages of the COVID-19 related diseases, and with forthright comparisons to other therapeutic combinations. Preliminary results could be available in a few weeks for certain of these studies. Side effects of current oral doses of HCQ are significant, but they are not expected to occur at short term (5), except for the rare cardiac risk related to a prolonged QT interval (6), although the safety profile of HCQ is still unknown in the particular population of the old subjects who are particularly at risk of serious COVID-19 diseases.

Thus, there is still an urgent need to find a new treatments of Severe Acute Respiratory Syndrome-Corona Virus (SARS-CoV) infection, including alternative administration methods.

### SUMMARY OF THE INVENTION:

The invention relates to methods and compositions for treatment of infection with Coronaviruses. In particular, the invention is claimed by the claims.

### DETAILED DESCRIPTION OF THE INVENTION:

The current COVID-19 pandemic is predicted to last several additional months, further increasing mortality rates, especially in the more fragile populations. In addition, at a longer term, there are major concerns for possible resurgences and for the potential morbidity due to fibrotic sequels.

At the moment, none of the current clinical trials are conducted with an aerosol delivery of anti-viral agents, whereas COVID-19 mainly set and presumable replicate in upper and especially lower airways. In a general way, a pulmonary therapeutic target may be reached with aerosols in a much more rapid and effective way than with a systemic oral or intravenous administration, including for HCQ for which the aerosol delivery was successfully tested in animals and has given evidence of a rapid and potent anti-inflammatory effect on the lung. The inventors aim at developing a new therapeutic approach based on the local delivery of hydroxychloroquine (HCQ) or its metabolite (e.g. Desethylhydroxychloroquine (DHCQ)) into the lung by aerosolization.

Accordingly, the first object of the present invention relates to a method of treating a lung infection in a patient in need thereof comprising administering to the patient's lungs a therapeutically effective amount of hydroxychloroquine (HCQ) or its metabolite (e.g. Desethylhydroxychloroquine (DHCQ)) by aerosolization.

In particular, the present invention also to a method of treating Severe Acute Respiratory Syndrome-Corona Virus (SARS-CoV) in a patient in need thereof comprising administering to the patient's lungs a therapeutically effective amount of hydroxychloroquine (HCQ) or its metabolites by aerosolization.

In particular, the present invention also relates to a method of treating Severe Acute Respiratory Syndrome-Corona Virus (SARS-CoV) in a patient in need thereof comprising administering to the patient's lungs a therapeutically effective amount of hydroxychloroquine (HCQ) by aerosolization.

In particular, the present invention also relates to a method of treating Severe Acute Respiratory Syndrome-Corona Virus (SARS-CoV) in a patient in need thereof comprising administering to the patient's lungs a therapeutically effective amount of hydroxychloroquine (HCQ) and/or Desethylhydroxychloroquine (DHCQ) by aerosolization.

As used herein, the term "lung infection" has its general meaning in the art and means the invasion of lung tissues of a patient by disease-causing microorganisms, their multiplication and the reaction of lung tissues to these microorganisms and the toxins that they produce.

In some embodiments, the patient suffers from a chronic lung infection. As used herein, the term "chronic infection" refers to a long-term infection which may be an apparent, unapparent or latent infection.

In some embodiments, the patient suffers from an acute lung infection. As used herein, the term "acute lung infection" has its general meaning in the art and refers to a disease of the lungs characterized by inflammation and consolidation followed by resolution and caused by infection from viruses, fungi, or bacteria. The term is also known as "pneumonia". Typically acute lung infection is associated with lung inflammation that is the rapid onset of progressive malfunction of the lungs, and is usually associated with the malfunction of other organs due to the inability to take up oxygen.

As used herein the term "patient" refers to a mammalian to which the present invention may be applied. Typically said mammal is a human, but may concern other mammals such as primates, dogs, cats, pigs, sheep, cows. In particular, the term "patient" refers to a mammalian patient, such as a human, who is confirmed to have a lung infection or who may be classified as having a probable or suspected case of a lung infection based on epidemiological factors. Patients include those who are diagnosed with a lung infection, those who test positive for infection by an infectious agent (pathogen) associated with a lung infection (e.g., SARS-CoV), those who are suspected of having a lung infection based on epidemiological factors, or those who are at an imminent risk of contracting a lung infection (e.g., one who has been exposed or will likely be exposed to a lung infection in the near future).

In some embodiments, the subject is a human infant. In some embodiments, the subject is a human child. In some embodiments, the subject is a human adult. In some embodiments, the subject is an elderly human. In some embodiments, the subject is a premature human infant.

As used herein, the term "treatment" or "treat" refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a patient having a medical disorder or who ultimately may acquire the disorder, in order to prevent, cure, delay the onset of, reduce the severity of, or ameliorate one or more symptoms of a disorder or recurring disorder, or in order to prolong the survival of a patient beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular interval, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

In some embodiments, the lung infection is a viral infection, such viral pneumonia. In some embodiments, the viral infection is caused by a virus selected from the group consisting of influenza virus (e.g., Influenza virus A, Influenza virus B), respiratory syncytial virus, adenovirus, metapneumovirus, cytomegalovirus, parainfluenza virus (e.g., hPIV-1, hPIV-2, hPIV-3, hPIV-4), rhinovirus, coxsackie virus, echo virus, herpes simplex virus, coronavirus (SARS-coronavirus such as SARS-CoV1 or SARS-CoV 2), and smallpox. In some embodiments, the viral lung infection may be due to a member of the *Pneumoviridae*, *Paramyxoviridae* and/or *Coronaviridae* families are in particular selected from the group consisting of upper and lower respiratory tract infections due to: human respiratory syncytial virus (hRSV), type A and type B, human metapneumovirus (hMPV) type A and type B; parainfluenza virus type 3 (PIV-3), measles virus, endemic human coronaviruses (HCoV-229E, -NL63, -OC43, and -HKU1), severe acute respiratory syndrome (SARS) and Middle-East respiratory syndrome (MERS) coronaviruses. In particular, the method of the present invention is suitable for the treatment of Severe Acute Respiratory Syndrome (SARS).

In particular, the method of the present invention is suitable for the treatment of Severe Acute Respiratory Syndrome-Corona Virus (SARS-CoV). More particularly, the method of the present invention is suitable for the treatment of SARS-CoV 1 or SARS-CoV 2.

In some embodiment, the SARS-CoV is COVID-19. The method of the present invention is suitable for the treatment of COVID-19.

As used herein, the term "severe acute respiratory syndrome coronavirus" or "SARS-coronavirus" is a strain of virus that causes severe acute respiratory syndrome. It is an enveloped, positive-sense, single-stranded RNA virus which infects the epithelial cells within the lungs. The virus enters the host cell by binding to the ACE2 receptor. SARS-coronavirus comprises SARS-CoV 1 or SARS-CoV 2.

As used herein, the term "severe acute respiratory syndrome coronavirus 1" or SARS-CoV-1) is the coronavirus responsible for the SARS epidemic from 2002 to 2004. It is a strain of the coronavirus species SARSr-CoV. This infectious agent is said to have appeared in November 2002 in Guangdong province, China. Between November 1, 2002 and August 31, 2003, the virus would have infected 8,096 people in thirty countries, causing 774 deaths, mainly in China, Hong Kong, Taiwan, and Southeast Asia. It is a single-stranded RNA virus of positive polarity belonging to the genus betacoronavirus

As used herein, the term "severe acute respiratory syndrome coronavirus 2" or SARS-CoV-2) is a positive-sense single-stranded RNA virus. It causes coronavirus disease 2019 (COVID-19), a respiratory illness. SARS-CoV-2 is a member of the subgenus *Sarbecovirus* (beta-CoV lineage B). Its RNA sequence is approximately 30,000 bases in length. SARS-CoV-2 is unique among known betacoronaviruses in its incorporation of a polybasic cleavage site, a characteristic known to increase pathogenicity and transmissibility in other viruses.

In some embodiments, the patient suffers from COVID-19.

In some embodiments, the lung infections include but are not limited to pneumonia (including community-acquired pneumonia, nosocomial pneumonia (hospital-acquired pneumonia, HAP; health-care associated pneumonia, HCAP), ventilator-associated pneumonia (VAP)), ventilator-associated trachebronchitis (VAT), and bronchitis.

As used herein, the term "hydroxychloroquine" or "HCQ" has its general meaning in the art and refers to 2-[[4-[(7-Chloro-4-quinolyl) amino] pentyl] ethylamino] ethanol sulfate (1:1). Methods of synthesis for hydroxychloroquine are disclosed in U.S. Pat. No. 2,546,658, herein incorporated by reference. The HCQ has the following structure:

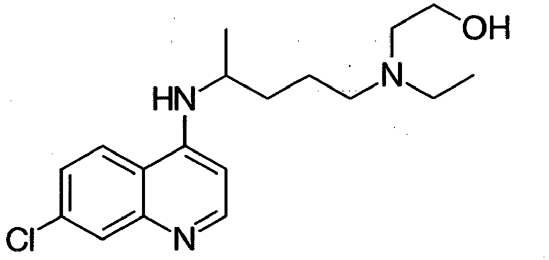

HCQ has similar pharmacokinetics to chloroquine, with rapid gastrointestinal absorption and elimination by the kidneys. Cytochrome P450 enzymes (CYP2D6, 2C8, 3A4 and 3A5). HCQ is metabolized into three major metabolites, desethyl-chloroquine (DCQ), bisdesethylchloroquine (BDCQ), and N-desethylhydroxychloroquine (DHCQ).

In some embodiment, the metabolites of HCQ are desethyl-chloroquine (DCQ), bisdesethylchloroquine (BDCQ), and N-desethylhydroxychloroquine (DHCQ).

The present invention also relates to a method of treating Severe Acute Respiratory Syndrome-Corona Virus (SARS-CoV) in a patient in need thereof comprising administering to the patient's lungs a therapeutically effective amount of N-desethylhydroxychloroquine" (DHCQ) by aerosolization.

As used herein, the term "N-desethylhydroxychloroquine" or "DHCQ" has its general meaning in the art and has the following structure:

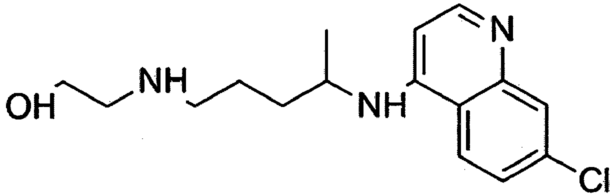

The present invention also relates to a method of treating Severe Acute Respiratory Syndrome-Corona Virus (SARS-CoV) in a patient in need thereof comprising administering to the patient's lungs a therapeutically effective amount of bisdesethylchloroquine (BDCQ) by aerosolization.

As used herein, the term "bisdesethylchloroquine" or "BDCQ" has its general meaning in the art, refers to 4-N-(7-chloroquinolin-4-yl)pentane-1,4-diamine and has the following structure:

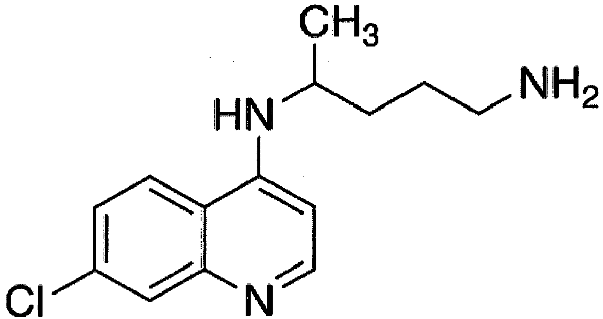

The present invention also relates to a method of treating Severe Acute Respiratory Syndrome-Corona Virus (SARS-CoV) in a patient in need thereof comprising administering to the patient's lungs a therapeutically effective amount of desethyl-chloroquine (DCQ) by aerosolization.

As used herein, the term "desethyl-chloroquine" or "DCQ" has its general meaning in the art, refers 4-N-(7-chloroquinolin-4-yl)-1-N-ethylpentane-1,4-diamine and has the following structure:

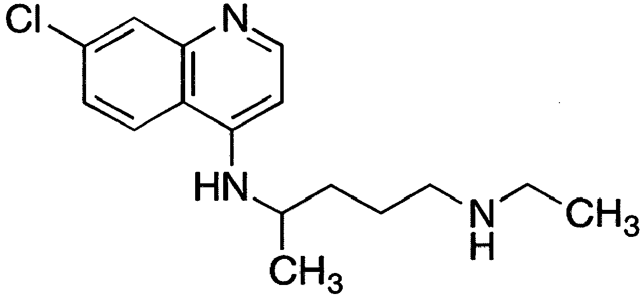

As used herein, the expression "effective amount" means an amount of HCQ used in the present invention sufficient to result in the desired therapeutic response i.e. the treatment of the lung infection. It will be understood that the total daily usage of the compounds and compositions of the present invention will be decided by the attending physician within the scope of sound medical judgment. The specific therapeutically effective dose level for any particular subject will depend upon a variety of factors including the age, body weight, general health, sex and diet of the subject; the time of administration, route of administration, and rate of excretion of the specific compound employed; the duration of the treatment; drugs used in combination or coincidental with the specific polypeptide employed; and like factors well known in the medical arts. For example, it is well known within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the active ingredient for the symptomatic 20 adjustment of the dosage ' to the subject to be treated. A medicament typically contains from about 0.01 mg to about 500 mg of the active ingredient, typically from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day. In particular, the daily dosage of the products of the invention (i.e. HCQ or DHCQ) may be varied over a wide range from 1 a 3 mg/kg per adult per day.

The product of the invention (i.e. HCQ or DHCQ) may be administered once, twice, three times, four times, five times per day.

As used herein the terms "administering" or "administration" refer to the act of injecting or otherwise physically delivering a substance as it exists outside the body (e.g., HCQ and/or DHCQ) into the subject, such as by nasal, oral, mucosal, intradermal, intravenous, subcutaneous, intramuscular delivery and/or any other method of physical delivery described herein or known in the art. When a disease, or a symptom thereof, is being treated, administration of the substance typically occurs after the onset of the disease or symptoms thereof. When a disease or symptoms thereof, are being prevented, administration of the substance typically occurs before the onset of the disease or symptoms thereof. In a particular embodiment, the subject is administered by aerosolization.

In some embodiment, the hydroxychloroquine (HCQ) is administered by aerosolization.

In some embodiment, the N-desethylhydroxychloroquine (DHCQ) is administered by aerosolization.

In some embodiment, the desethylchloroquine (DCQ) is administered by aerosolization.

In some embodiment, the bisdesethylchloroquine (BDCQ) is administered by aerosolization.

In some embodiment, the hydroxychloroquine (HCQ) is administered by oral.

In some embodiment, the N-desethylhydroxychloroquine (DHCQ) is administered by oral.

In some embodiment, the desethylchloroquine (DCQ) is administered by oral.

In some embodiment, the bisdesethylchloroquine (BDCQ) is administered by oral.

As used herein, the term "aerosolization" refers to a process whereby a liquid or powder or a vial formulation is converted to an aerosol. Typically, aerosolization is performed by a nebulizer.

As used herein, the term "nebulizer" or "aerosol generator" has its general meaning in the art and refers to a device that converts a liquid or powder or a vial into an aerosol of a size that can be inhaled into the respiratory tract. Pneumonic, ultrasonic, electronic nebulizers, e.g., passive electronic mesh nebulizers, active electronic mesh nebulizers and vibrating mesh nebulizers are amenable for use with the invention if the particular nebulizer emits an aerosol with the required properties, and at the required output rate. The process of pneumatically converting a bulk liquid into small droplets is called atomization. The operation of a pneumatic nebulizer requires a pressurized gas supply as the driving force for liquid atomization. Ultrasonic nebulizers use electricity introduced by a piezoelectric element in the liquid reservoir to convert a liquid into respirable droplets. Various types of nebulizers are described in Respiratory Care, Vol. 45, No. 6, pp. 609-622 (2000), the disclosure of which is incorporated herein by reference in its entirety. The terms "nebulizer" and "aerosol generator" are used interchangeably throughout the specification. "Inhalation device", "inhalation system" and "atomizer" are also used in the literature interchangeably with the terms "nebulizer" and "aerosol generator".

For instance, the device can include a ventilator, optionally in combination with a mask, mouthpiece, mist inhalation apparatus, and/or a platform that guides users to inhale correctly and automatically deliver the drug (i.e. the HCQ or DHCQ) at the right time in the breath. Representative aerosolization devices that can be used in accordance with the methods of the present invention include but are not limited to those described in U.S. Patent Nos. 6,357,671 ; 6,354,516; 6,241 ,159; 6,044,841 ; 6,041 ,776; 6,016,974; 5,823,179; 5,797,389; 5,660,166; 5,355,872; 5,284,133; and 5,277,175 and U.S. Published Patent Application Nos. 20020020412 and 20020020409.

Using a jet nebulizer, compressed gas from a compressor or hospital airline is passed through a narrow constriction known as a jet. This creates an area of low pressure, and liquid medication from a reservoir is drawn up through a feed tube and fragmented into droplets by the air stream. Only the smallest drops leave the nebulizer directly, while the majority impact on baffles and walls and are returned to the reservoir. Consequently, the time required to perform jet nebulization varies according to the volume of the composition to be nebulized, among other factors, and such time can readily be adjusted by one of skill in the art.

A metered dose inhalator (MDI) can be used to deliver a composition of the invention in a more concentrated form than typically delivered using a nebulizer. For optimal effect, MDI delivery systems require proper administration technique, which includes coordinated actuation of aerosol delivery with inhalation, a slow inhalation of about 0.5-0.75 liters per second, a deep breath approaching inspiratory capacity inhalation, and at least 4 seconds of breath holding. Pulmonary delivery using a MDI is convenient and suitable when the treatment benefits from a relatively short treatment time and low cost.

Aerosolized HCQ or DHCQ of the invention comprise droplets that are a suitable size for efficient delivery within the lung. Preferably, the formulation is effectively delivered to lung bronchi, more preferably to bronchioles, still more preferably to alveolar ducts, and still more preferably to alveoli. Thus, aerosol droplets are typically less than about 15 µm in diameter, and preferably less than about 10 µm in diameter, more preferably less than about 5 µm in diameter, and still more preferably less than about 2 µm in diameter. For efficient delivery to alveolar bronchi of a human subject or for efficient delivery to the upper airway: Oropharynx and tracheobronchial zones of a human subject, an aerosol composition preferably comprises droplets having a diameter of about 1 µm to about 5 µm. Droplet size can be assessed using techniques known in the art, for example cascade, impaction, laser diffraction, and optical pattemation. See McLean et al. (2000) Anal Chem 72:4796-804, Fults et al. (1991) J Pharm Pharmacol 43:726-8, and Vecellio None et al. (2001) J Aerosol Med 14:107-14.

The compositions of the present invention may comprise one or more pharmaceutically acceptable excipients, in particular selected from the group of an HFC/HFA propellant, a cosolvent, a bulking agent, a non-volatile component, a buffer/pH adjusting agent, a surfactant, a preservative, a complexing agent, or combinations thereof. Suitable propellants are those which, when mixed with the solvent(s), form a homogeneous propellant system in which a therapeutically effective amount of HCQ or DHCQ can be dissolved. The HFC/HFA propellant must be toxicologically safe and must have a vapor pressure which is suitable to enable the HCG or DHCQ to be administered via a pressurized MDI. According to the present invention, the HFC/HFA propellants may comprise, one or more of 1,1,1,2-tetrafluoroethane (HFA-134(a)) and 1,1,1,2,3,3,3,-heptafluoropropane (HFA-227), HFC-32 (difluoromethane), HFC-143(a) (1,1,1-trifluoroethane), HFC-134 (1,1,2,2-tetrafluoroethane), and HFC-152a (1,1-difluoroethane) or combinations thereof and such other propellants which may be known to the person having a skill in the art.

The HCQ and/or the DHCQ administered by aerosolization as described above is combined with HCQ and/or the DHCQ administered by oral

Accordingly, in a second aspect, the invention relates to i) HCQ and/or the DHCQ administered by aerosolization and ii) HCQ and/or the DHCQ administered by oral used as a combined preparation for treating COVID-19 in a subject.

Accordingly, in a second aspect, the invention relates to i) HCQ administered by aerosolization and ii) HCQ administered by oral used as a combined preparation for treating SARS-CoV (e.g COVID-19) in a subject.

Accordingly, in a second aspect, the invention relates to i) DHCQ administered by aerosolization and ii) DHCQ administered by oral used as a combined preparation for treating SARS-CoV (e.g COVID-19) in a subject.

Accordingly, in a second aspect, the invention relates to i) HCQ administered by aerosolization and ii) DHCQ administered by oral used as a combined preparation for treating SARS-CoV (e.g COVID-19) in a subject.

Accordingly, in a second aspect, the invention relates to i) DHCQ administered by aerosolization and ii) HCQ administered by oral used as a combined preparation for treating SARS-CoV (e.g COVID-19) in a subject.

As used herein, the terms "combined treatment", "combined therapy" or "therapy combination" refer to a treatment that uses more than one medication. The combined therapy may be dual therapy or bi-therapy.

As used herein, the term "administration simultaneously" refers to administration of 2 active ingredients by the same route and at the same time or at substantially the same time. The term "administration separately" refers to an administration of 2 active ingredients at the same time or at substantially the same time by different routes. The term "administration sequentially" refers to an administration of 2 active ingredients at different times, the administration route being identical or different.

The invention will be further illustrated by the following examples. However, these examples and figures should not be interpreted in any way as limiting the scope of the present invention.

### EXAMPLE:

As the previous severe acute respiratory syndrome coronavirus (SARS-CoV), Covid-19 uses the same cellular entry receptor, the angiotensin converting enzyme 2 (ACE2), which is present in high quantity in the lower respiratory tract of humans (11), especially on type II alveolar cells (AT2) (12). COVIDS affects firstly the respiratory mucosa and it might further invade other organs, triggering a series of immune responses and possibly leading to a "cytokine storm production", with an immune response out of control, and ultimately leading to critical conditions in certain COVID patients (11). Type II alveolar cells (AT2), which are involved in the regeneration of the pulmonary surfactant, xenobiotic metabolism, the regeneration of alveolar epithelium, constitute the main viral replication site in the lung (12). However, based on studies of receptor ACE2 expression, it has been considered that other cell types are vulnerable to COVID infection, especially myocardial cells, proximal tubule cells of the kidney, ileum and esophagus epithelial cells, and bladder urothelial cells (12).

Studies conducted *in vitro* at a cellular level have shown that HCQ is a highly potent antiviral drug, including for COVID (1,2). If administered as an aerosol in patients affected by COVID related lung diseases, HCQ could reach the same pulmonary sites as HCQ circulating in the blood. This consideration is supported by results from scintigraphic examinations carried out in COVID-19 patients and showing that the pulmonary regions reached by blood radiotracers are also reached by radioactive aerosols (Data not shown). HCQ and DHCQ are water-soluble, they do not aggregate when dissolved in water. According to a molecular modeling work (Data not shown), if HCQ/DHCQ are administered as an aerosol, they could reach the epithelial cells of the airways, they could diffuse through pulmonary surfactant to enter within type II pneumocytes, an expected site of therapeutic effects. This pathway is short and direct, fairly close to that of the HCQ administered in cell culture, and in any case much faster than the complex kinetics leading to reach pulmonary targets after an oral administration.

### REFERENCES:

Throughout this application, various references describe the state of the art to which this invention pertains. The disclosures of these references are hereby incorporated by reference into the present disclosure.
1. Yao X, Ye F, Zhang M, et al. In Vitro Antiviral Activity and Projection of Optimized Dosing Design of Hydroxychloroquine for the Treatment of Severe Acute Respiratory Syndrome Coronavirus 2 (SARS-CoV-2). Clin Infect Dis. 2020 Mar 9. doi: 10.1093/cid/ciaa237. [Epub ahead of print].
2. Wang M, Cao R, Zhang L, et al. Remdesivir and chloroquine effectively inhibit the recently emerged novel coronavirus (2019-nCoV) in vitro. Cell Res. 2020;30(3):269-271.
3. Gao J, Tian Z, Yang X. Breakthrough: Chloroquine phosphate has shown apparent efficacy in treatment of COVID-19 associated pneumonia in clinical studies. Biosci Trends 2020. doi: 10.5582/bst.2020.01047. [Epub ahead of print]
4. Gautret P, Lagier JC, Parola P, et al. Hydroxychloroquine and azithromycin as a treatment of COVID-19: results of an open-label non-randomized clinical trial. Int J Antimicrob Agents. 2020. doi: 10.1016/j.ijantimicag.2020.105949. [Epub ahead of print].
5. Zhou D, Dai SM, Tong Q. COVID-19: a recommendation to examine the effect of hydroxychloroquine in preventing infection and progression. J Antimicrob Chemother. 2020. doi: 10.1093/jac/dkaa114. [Epub ahead of print]
6. O'Laughlin JP, Mehta PH, Wong BC. Life Threatening Severe QTc Prolongation in Patient with Systemic Lupus Erythematosus due to Hydroxychloroquine. Case Rep Cardiol. 2016;2016:4626279.
7. Hanley B, Lucas SB, Youd E, et al. Autopsy in suspected COVID-19 cases.J Clin Pathol. 2020. doi: 10.1136/jclinpath-2020-206522. [Epub ahead of print].
8. Tian S, Hu W, Niu L, Liu H, et al. Pulmonary Pathology of Early-Phase 2019 Novel Coronavirus (COVID-19) Pneumonia in Two Patients With Lung Cancer. J Thorac Oncol. 2020. doi: 10.1016/j.jtho.2020.02.010. [Epub ahead of print]
9. He F, Deng Y, Li W. Coronavirus disease 2019: What we know? J Med Virol. 2020 Mar 14. doi:
10.1002/jmv.25766. [Epub ahead of print]10.Qin C, Liu F, Yen TC, et al. 18 F-FDG PET/CT findings of COVID-19: a series of four highly suspected cases. Eur J Nucl Med Mol Imaging. 2020;47(5):1281-1286.
11. Guo YR, Cao QD, Hong ZS, et al. The origin, transmission and clinical therapies on coronavirus disease 2019 (COVID-19) outbreak - an update on the status. Mil Med Res. 2020;7(1):11.
12. Zou X, Chen K, Zou J, et al. Single-cell RNAseq data analysis on the receptor ACE2 expression reveals the potential risk of different human organs vulnerable to 2019-nCoV infection. Front Med. 2020. doi: 10.1007/s11684-020-0754-0. [Epub ahead of print]
13. Coates AL, Green M, Leung K, et al. The challenges of quantitative measurement of lung deposition using 99mTc-DTPA from delivery systems with very different delivery times. J Aerosol Med. 2007;20(3):320-30.
14. Sharma P, Yi R, Nayak AP, et al. Bitter Taste Receptor Agonists Mitigate Features of Allergic Asthma in Mice. Sci Rep. 2017;7:46166.

## Claims

1. A method of treating Severe Acute Respiratory Syndrome-Corona Virus (SARS-CoV) in a patient in need thereof comprising administering to the patient's lungs a therapeutically effective amount of hydroxychloroquine (HCQ) or its metabolites by aerosolization.

2. The method according to claim 1 wherein the SARS-CoV is COVID-19.

3. The method according to claim 1 wherein the metabolite is N-desethylhydroxychloroquine (DHCQ).

4. The method according to claim 1 wherein the metabolite is desethylchloroquine (DCQ).

5. The method according to claim 1 wherein the metabolite is bisdesethylchloroquine (BDCQ).

6. The method according to claim 1 wherein the HCQ administered by aerosolization is combined with HCQ administered by oral.

7. The method according to claim 3 wherein the DHCQ administered by aerosolization is combined with HCQ administered by oral.
